# EUROPEAN PATENT SPECIFICATION

(11) **EP 4 409 043 B1**
(45) Date of publication and mention of the grant of the patent: **27.08.2025**
(21) Application number: 22799869.7
(22) Date of filing: 29.09.2022
(51) Int. Cl.: C12Q 1/6886, C12Q 1/689

(54) **FAECAL MICROBIOTA SIGNATURE FOR PANCREATIC CANCER**
FÄKALIENMIKROBIOTASIGNATUR FÜR BAUCHSPEICHELDRÜSENKREBS
SIGNATURE MICROBIOTE FÉCALE POUR LE CANCER DU PANCRÉAS

(30) Priority: 29.09.2021 EP 21382876
(43) Date of publication of application: 07.08.2024
(73) Proprietor: Fundación del Sector Público Estatal Centro Nacional de Investigaciones Oncológicas Carlos III (F.S.P. CNIO), 28029 Madrid (ES); European Molecular Biology Laboratory, 69117 Heidelberg (DE)
(72) Inventor: MALATS RIERA, Nuria, 28029 Madrid (ES); BORK, Peer, 69117 Heidelberg (DE); KARTAL, Ece, 69117 Heidelberg (DE); MOLINA MONTES, Esther, 28029 Madrid (ES); RODRÍGUEZ, Sandra, 28029 Madrid (ES); ESTUDILLO, Lidia, 28029 Madrid (ES); REAL, Francisco Xavier, 28029 Madrid (ES); SCHMIDT, Thomas S.B., 79106 Freiburg (DE); ZELLER, Georg, 69118 Heidelberg (DE); WIRBEL, Jakob, 69115 Heidelberg (DE); MAISTRENKO, Oleksandr M., 1797 TA Den Hoorn (Texel) (NL)
(74) Representative: Clarke, Modet y Cía., S.L.
(86) International application number: PCT/EP2022/077087
(87) International publication number: WO 2023/052486

(56) References cited:
- WO-A1-2018/145082
- US-A1- 2021 260 092
- REN ZHIGANG ET AL: "Gut microbial profile analysis by MiSeq sequencing of pancreatic carcinoma patients in China", ONCOTARGET, vol. 8, no. 56, 10 November 2017 (2017-11-10), pages 95176 - 95191, XP055902650, DOI: 10.18632/oncotarget.18820
- HALF ELIZABETH ET AL: "Fecal microbiome signatures of pancreatic cancer patients", vol. 9, no. 1, 1 December 2019 (2019-12-01), pages 16801, XP055902653, Retrieved from the Internet <URL:https://www.ncbi.nlm.nih.gov/pmc/articles/PMC6856127/pdf/41598_2019_Article_53041.pdf> DOI: 10.1038/s41598-019-53041-4
- KOHI SHIRO ET AL: "Alterations in the Duodenal Fluid Microbiome of Patients With Pancreatic Cancer", CLINICAL GASTROENTEROLOGY AND HEPATOLOGY, ELSEVIER, AMSTERDAM, NL, vol. 20, no. 2, 5 November 2020 (2020-11-05), XP086923207, ISSN: 1542-3565, [retrieved on 20201105], DOI: 10.1016/J.CGH.2020.11.006
- CONSTANTINOS P. ZAMBIRINIS ET AL: "Pancreatic Cancer, Inflammation, and Microbiome", CANCER JOURNAL, vol. 20, no. 3, 1 January 2014 (2014-01-01), US, pages 195 - 202, XP055227166, ISSN: 1528-9117, DOI: 10.1097/PPO.0000000000000045
- LI JENNY JING ET AL: "The role of microbiome in pancreatic cancer", CANCER METASTASIS, KLUWER ACADEMIC PUBLISHERS, DORDRECHT, NL, vol. 40, no. 3, 28 August 2021 (2021-08-28), pages 777 - 789, XP037603779, ISSN: 0167-7659, [retrieved on 20210828], DOI: 10.1007/S10555-021-09982-2

## Description

### FIELD OF THE INVENTION

The present invention relates to a method for diagnosing pancreatic cancer based on a faecal microbiota signature with high specificity for pancreatic cancer.

### BACKGROUND OF THE INVENTION

Pancreatic ductal adenocarcinoma (PDAC) is the most frequent form of pancreatic cancer that poses an increasing disease burden worldwide.

The high lethality of PDAC is a consequence of both late diagnosis and limited therapeutic options. The symptoms are unspecific and often emerge only during late disease stages, at which point tumors can be non-resectable.

PDAC has a complex etiology, with established risk factors that include age, chronic pancreatitis, diabetes mellitus, obesity, asthma, blood group and lifestyle. The role of these risk factors in PDAC etiology may also be complemented or mediated by alterations in the microbiome, being associated with disease progression and long-term survival in PDAC patients.

Half, E. et al., Sci. Rep. 9, 16801 (2019), relates to gut microbiome alterations in pancreatic cancer and their potential to serve as biomarkers.

WO2018145082A1 relates to the treatment and diagnosis of pancreatic cancer based on cecal, ileal, colonic and faecal microbiota and particularly focuses on different signatures in the gastrointestinal microbiota.

Ren, Z. et al., Oncotarget Vol. 8, No. 56, pp: 95176-95191 (2017), relates to gut microbial markers for pancreatic cancer diagnosis.

There is a still a need for sensitive, specific non-invasive and affordable tests for an early detection of PDAC that could therefore improve survival outcomes.

### SUMMARY OF THE INVENTION

The present invention relates to a method for diagnosing pancreatic cancer based on a faecal microbiota signature with high specificity for pancreatic cancer.

In a first aspect, the present invention relates to a method for diagnosing pancreatic cancer in a subject comprising:
a. determining the abundance of at least the following Microbiome species: *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii, and Bacteroides finegoldii* in a faecal sample from said subject, and:
   b. identifying the subject as:
   i. having pancreatic cancer when the profile of abundance for the analysed species corresponds to a profile of abundance of the analysed species of a pancreatic cancer reference group, or
   ii. not having pancreatic cancer when the profile of abundance for the analysed species corresponds to a profile of abundance of the analysed species of a reference group not having pancreatic cancer.

In a second aspect, the present invention relates to a kit of parts comprising specific primers and optionally also specific probes for the detection/quantification of the microbial presence/abundance of the species *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii, and Bacteroides finegoldii.*

### DESCRIPTION OF THE INVENTION

Nowadays there currently exists no screening tool for PDAC in the clinic, in particular for the detection of early disease stages. The inventors have found a microbiome signature that emerges early during the disease's progression and that the faecal microbiome is very useful for the early detection of PDAC. In particular, the present invention provides a method that robustly and accurately predicts PDAC solely based on characteristic faecal microbial species. Furthermore, the combination of the claimed microbiome classifiers with the serum CA19-9 level significantly enhances the accuracy of PDAC detection. In summary, the described faecal microbiome signature enables robust PDAC detection with high disease specificity, complementary to existing markers, providing a cost-effective PDAC screening and monitoring method.

In a preferred embodiment of the first aspect, the method further comprises analysing the abundance of at least one of the following species: *Faecalibacillus intestinalis, Streptococcus oralis, Streptococcus gordonii*/c*ristatus, Romboutsia timonensis, Ruminococcus callidus, Atopobium parvulum, Fusobacterium periodonticum, Fusobacterium hwasookii*/*nucleatum, Phascolarctobacterium faecium, Dialister pneumosintes, Prevotella sp. CAG:279* [meta-mOTU-v2.5 12279] and *Duodenibacillus massiliensis* in said faecal sample.

In a preferred embodiment of the first aspect, the method further comprises analysing the abundance of the following species *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii, Bacteroides finegoldii, Faecalibacillus intestinalis, Streptococcus oralis, Streptococcus gordonii*/c*ristatus, Romboutsia timonensis, Ruminococcus callidus, Atopobium parvulum, Fusobacterium periodonticum, Fusobacterium hwasookii*/*nucleatum, Phascolarctobacterium faecium, Dialister pneumosintes, Prevotella sp. CAG:279* [meta-mOTU-v2.5 12279] and *Duodenibacillus massiliensis*

In a preferred embodiment of the first aspect, the method consists essentially in:
c. analysing the abundance of at least the following Microbiome species: *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii and Bacteroides finegoldii,* in said faecal sample. and:
d. identifying the subject as:
i. having pancreatic cancer when the profile of abundance for the analysed species corresponds to a profile of abundance of the analysed species of a pancreatic cancer reference group, or
ii. not having pancreatic cancer when the profile of abundance for the analysed species corresponds to a profile of abundance of the analysed species of a reference group not having pancreatic cancer.

*Veillonella sp.* [meta-mOTU-v2.5 13135] and *Prevotella sp. CAG:279* [meta-mOTU-v2.5 12279] are a specific species that have not been named yet. The skilled person would know how to analyse the abundance of this species in a faecal sample by using the markers publicly disclosed in the mOTU database, particularly in meta-mOTU-v2.5 13135 and meta-mOTU-v2.5 12279 dated 17^{th} August 2019.

In a preferred embodiment of the first aspect, the method further comprises analysing the levels of carbohydrate antigen (CA) 19-9 in a serum sample from said subject.

As used herein, the expression "CA 19-9" refers to an antigen which is routinely used to monitor PDAC progress and which is the only validated tumour marker for PDAC.

In a preferred embodiment of the first aspect, the subject is human.

In a preferred embodiment of the first aspect, the pancreatic cancer is in an early stage or a late stage of said pancreatic cancer.

As used herein, the expression "early stage" referred to pancreatic cancer refers to stages T1 or T2. T1: The tumour is in the pancreas only, and it is 2 centimetres (cm) or smaller in size. T2: The tumour is in the pancreas only, and it is larger than 2 cm but not larger than 4 cm. T3: The tumour is larger than 4 cm and extends beyond the pancreas. It does not involve the major arteries or veins near the pancreas. T4: The tumour extends beyond the pancreas into major arteries or veins near the pancreas. A T4 tumour cannot be completely removed with surgery.

In a preferred embodiment of the method of the first aspect, step (a) is performed by metagenomic sequencing, qPCR, ddPCR, 16S rRNA amplicon sequencing, or a combination thereof. Preferably, whole genome sequencing is performed using shotgun metagenomic sequencing.

As used herein, the term "qPCR" refers to quantitative PCR which is a quantitative method, also known as Real-Time PCR, to detect, characterize and quantify DNA in real-time.

As used herein, the term "ddPCR" refers to Droplet Digital PCR which is a method of dPCR, digital PCR, in which a 20 microliter sample reaction is divided into ~20,000 nanoliter-sized oil droplets through a water-oil emulsion technique, thermocycled to endpoint in a 96-well PCR plate, and fluorescence amplitude read for all droplets in each sample well in a droplet flow cytometer.

As used herein, the expression "16S rRNA amplicon sequencing" refers to a sequencing technique based on the amplification of the 16S rRNA gene.

In a preferred embodiment of the first aspect, the pancreatic cancer is pancreatic ductal adenocarcinoma (PDAC).

In a preferred embodiment of the first aspect, the method further comprises administering a pancreatic cancer treatment to the subject. Preferably, the treatment comprises administering to the subject an effective amount of the probiotic composition, surgery, radiotherapy, chemotherapy, immunotherapy, faecal material transplantation (FMT) and any combination thereof. The present invention also relates to a method of treating a subject afflicted from pancreatic cancer which has been diagnosed using the method of the first aspect, comprising administering to the subject an effective amount of the probiotic composition, surgery, radiotherapy, chemotherapy, immunotherapy, and any combination thereof. The present invention also relates to a method of treating a subject afflicted from pancreatic cancer comprising administering to the subject an effective amount of the probiotic composition, surgery, radiotherapy, chemotherapy, immunotherapy, and any combination thereof, and further comprising monitoring the response to said treatment by detecting/quantifying *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii, and Bacteroides finegoldii* in a faecal sample from said subject.

The present invention also relates to a method for diagnosing pancreatic cancer in a patient, said method comprising: (a) obtaining a faecal sample from a human patient; (b) detecting/quantifying Microbiome species *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii, and Bacteroides finegoldii* (and optionally also at least one of species: *Faecalibacillus intestinalis, Streptococcus oralis, Streptococcus gordonii*/*cristatus, Romboutsia timonensis, Ruminococcus callidus, Atopobium parvulum, Fusobacterium periodonticum, Fusobacterium hwasookii*/*nucleatum, Phascolarctobacterium faecium, Dialister pneumosintes, Prevotella sp. CAG:279 [meta-mOTU-v2.5 12279]*and *Duodenibacillus massiliensis)* in the faecal sample using whole-genome sequencing, qPCR, ddPCR, 16S rRNA amplicon sequencing, or a combination thereof, preferably using shotgun metagenomic sequencing or preferably using specific primers and optionally specific probes for the detection/quantification of said microbial species; and (c) diagnosing the patient with pancreatic cancer when the profile of abundance for the analysed species corresponds to a profile of abundance of the analysed species of a pancreatic cancer reference group. The present invention also relates to a method for diagnosing and treating pancreatic cancer in a patient, said method comprising: (a) obtaining a faecal sample from a human patient; (b) detecting/quantifying Microbiome species *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii, and Bacteroides finegoldii* (and optionally also at least one of species: *Faecalibacillus intestinalis, Streptococcus oralis, Streptococcus gordonii*/*cristatus, Romboutsia timonensis, Ruminococcus callidus, Atopobium parvulum, Fusobacterium periodonticum, Fusobacterium hwasookii*/*nucleatum, Phascolarctobacterium faecium, Dialister pneumosintes, Prevotella sp. CAG:279 [meta-mOTU-v2.5 12279]* and *Duodenibacillus massiliensis)* in the faecal sample; (c) diagnosing the patient with pancreatic cancer when the profile of abundance for the analysed species corresponds to a profile of abundance of the analysed species of a pancreatic cancer reference group; and (d) administering to the diagnosed patient an effective amount of the probiotic composition, surgery, radiotherapy, chemotherapy, immunotherapy, and any combination thereof. In a preferred embodiment, the method also comprises analysing the levels of carbohydrate antigen (CA) 19-9 in a serum sample from the patient.

In a preferred embodiment of the second aspect, the kit further comprises specific primers and optionally also specific probes for the detection/quantification of the microbial presence/abundance of at least one of species *Faecalibacillus intestinalis, Streptococcus oralis, Streptococcus gordonii*/c*ristatus, Romboutsia timonensis, Ruminococcus callidus, Atopobium parvulum, Fusobacterium periodonticum, Fusobacterium hwasookii*/*nucleatum, Phascolarctobacterium faecium, Dialister pneumosintes, Prevotella sp. CAG:279* [meta-mOTU-v2.5 12279] and *Duodenibacillus massiliensis.*

In a preferred embodiment of the second aspect, the kit further comprises specific primers and optionally also specific probes for the detection/quantification of the microbial presence/abundance of at least one control species.

As used herein, the term "control" refers to a particular microbial species whose abundance in faeces is constant. The control species is a species than is present in all humans. For example, *B. vulgatus* can be used as control species.

In a preferred embodiment of the second aspect, the kit comprises specific primers and optionally also specific probes for the detection/quantification of the microbial presence/abundance of at least the species *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii,* and *Bacteroides finegoldii,* and up to 100 species, preferably up to 90, 80, 70, 60, 50, 40, 30, 20, 15 species.

In a preferred embodiment of the second aspect, the kit comprises specific primers and optionally also specific probes for the detection/quantification of the microbial presence/abundance of at least the species *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii, Bacteroides finegoldii, Faecalibacillus intestinalis, Streptococcus oralis, Streptococcus gordonii*/c*ristatus, Romboutsia timonensis, Ruminococcus callidus, Atopobium parvulum, Fusobacterium periodonticum, Fusobacterium hwasookii*/*nucleatum, Phascolarctobacterium faecium, Dialister pneumosintes, Prevotella sp. CAG:279* [meta-mOTU-v2.5 12279] and *Duodenibacillus massiliensis,* and up to 100 species, preferably up to 90, 80, 70, 60, 50, 40, 30, 25 species.

In a preferred embodiment, the kit of the second aspect consists essentially in the means for the detection/quantification of the microbial presence/abundance of species *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii, and Bacteroides finegoldii* and, optionally, also for the detection/quantification of the microbial presence/abundance of species *Faecalibacillus intestinalis, Streptococcus oralis, Streptococcus gordonii*/c*ristatus, Romboutsia timonensis, Ruminococcus callidus, Atopobium parvulum, Fusobacterium periodonticum, Fusobacterium hwasookii*/*nucleatum, Phascolarctobacterium faecium, Dialister pneumosintes, Prevotella sp. CAG:279* [meta-mOTU-v2.5 12279], *Duodenibacillus massiliensis* and also optionally comprises means for detecting/quantifying control species in faecal samples. The kit may also comprise reactants useful for processing a faecal sample for the detection/quantification of bacterial species in said sample, as buffers, etc. In a preferred embodiment, the kit also includes reactants necessary to perform a qPCR, such as dNTPs, a polymerase, specific buffers, etc. In a preferred embodiment, the kit of the second aspect, further comprising means for quantifying antigen CA 19-9 in a serum sample. Said means for quantifying antigen CA 19-9 in a serum sample are well known by a skilled person and are, for example, at least one antibody against human antigen CA 19-9 which can be used in immunoassays such as enzyme-linked immunoassay (ELISA), Chemiluminescence immunoassay (CLIA) or radioimmunoassay (RIA) techniques.

In a preferred embodiment, the kit of the second aspect comprises specific means for the detection/quantification by qPCR, ddPCR, 16S rRNA amplicon sequencing, whole genome sequencing, for example means for shotgun metagenomic sequencing, of the species *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii, and Bacteroides finegoldii* and, optionally, also for the detection/quantification of the microbial presence/abundance of species *Faecalibacillus intestinalis, Streptococcus oralis, Streptococcus gordonii*/*cristatus, Romboutsia timonensis, Ruminococcus callidus, Atopobium parvulum, Fusobacterium periodonticum, Fusobacterium hwasookii*/*nucleatum, Phascolarctobacterium faecium, Dialister pneumosintes, Prevotella sp. CAG:279* [meta-mOTU-v2.5 12279], *Duodenibacillus massiliensis* and also optionally for detecting/quantifying control species in faecal samples.

In a preferred embodiment of the second aspect, the kit is used for the diagnosis of pancreatic cancer, preferably of PDAC.

Another aspect of the present invention relates to the use of the combination of species *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii, and Bacteroides finegoldii,* and optionally at least one of species *Faecalibacillus intestinalis, Streptococcus oralis, Streptococcus gordonii*/*cristatus, Romboutsia timonensis, Ruminococcus callidus, Atopobium parvulum, Fusobacterium periodonticum, Fusobacterium hwasookii*/*nucleatum, Phascolarctobacterium faecium, Dialister pneumosintes, Prevotella sp. CAG:279 [meta-mOTU-v2.5 12279]* and *Duodenibacillus massiliensis,* as biomarkers for the diagnosis and/or monitoring of pancreatic cancer, preferably PDAC. In a preferred embodiment, the combined use further comprises the use of antigen CA 19-9 as biomarker for the diagnosis and/or monitoring of pancreatic cancer, preferably PDAC.

Another aspect of the present invention relates to an *in vitro* method for monitoring pancreatic cancer, preferably PDAC, or for monitoring the response to a pancreatic cancer treatment. Another aspect of the present invention relates to the use of the kit of the second aspect for monitoring pancreatic cancer, preferably PDAC, or for monitoring the response to a pancreatic cancer treatment.

### As used herein, the term "comprises" also includes "consists". All embodiments of all aspects can be combined and all embodiments of all aspects are embodiments of the other aspects. BRIEF DESCRIPTION OF FIGURES

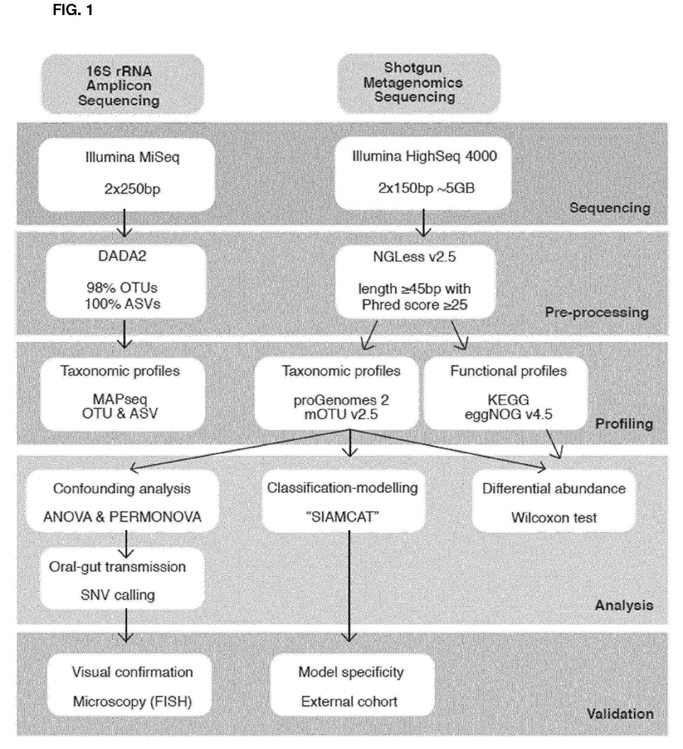

Figure 1: diagram of analysis steps for 16S rRNA amplicon sequencing data and for shotgun metagenomics sequencing data.

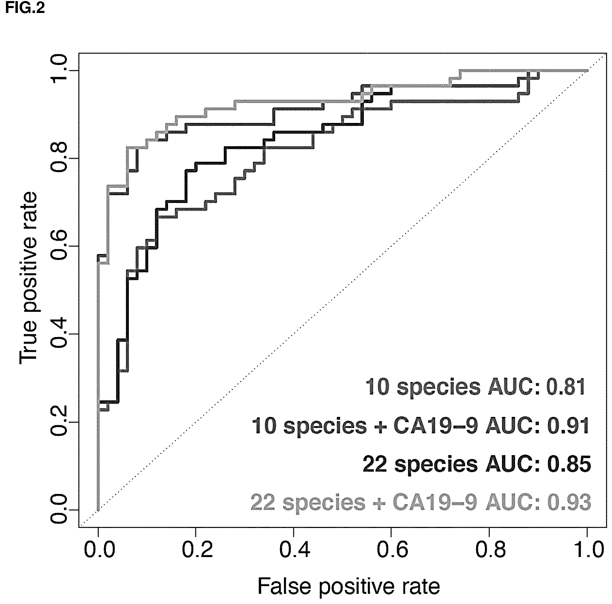

Figure 2. PDAC detection rate using the 10 species of claim 1 or the 22 species of claim 2, alone and in combination with CA 19-9 serum levels. Internal cross validation results of claim 1 and claim 2 models and combination with CA19-9 (using a threshold of 37ul/ml) with microbial features are shown as receiver operating characteristic (ROC) curve. True positive rates (TPR) are given as a percentage at 90% specificity cutoff.

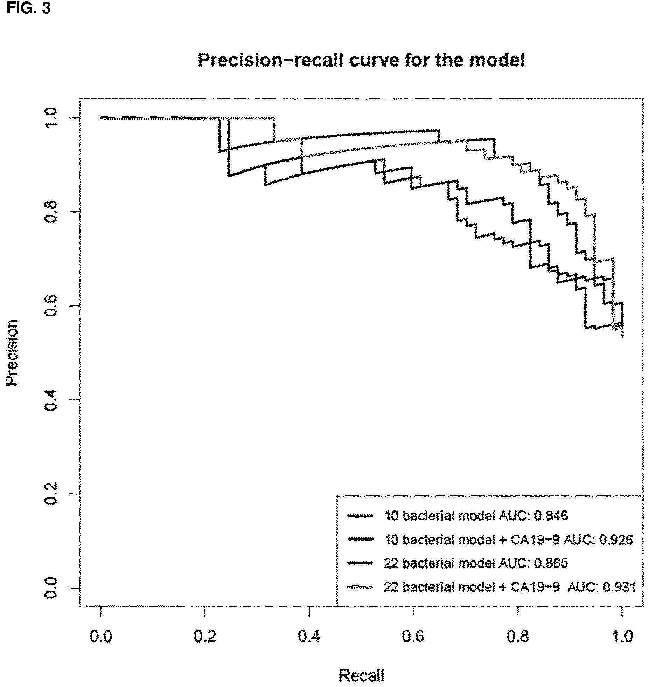

Figure 3. Precision-recall curve using the species of claim 1 and claim 2 alone and in combination with CA 19-9 serum levels. High score for precision relates to a low false positive rate, and recall relates to a low false negative rate, as well as returning a majority of all positive results (high recall). The stairstep area of the plot represent the relationship between precision and recall of claimed species with combination of CA19-9 levels.

### EXAMPLES

### Example 1. PDAC is associated with moderate shifts in microbiome composition when controlling for confounding factors.

57 newly diagnosed, treatment-naive PDAC patients, 29 chronic pancreatitis (CP) patients, and 50 age-, gender-, and hospital-matched controls were studied. Participants were prospectively recruited from two hospitals in Barcelona and Madrid, Spain, between 2016 and 2018, using the same standards. It was obtained faecal shotgun metagenomes for all subjects and salivary metagenomes for 45 patients with PDAC, 12 with CP, and 43 controls. The analysis workflow is detailed in Fig. 1.

As several PDAC risk factors, such as tobacco smoking, alcohol consumption, obesity, or diabetes, are themselves associated with microbiome composition, it was first sought to establish potential confounders of microbiome signatures in the study population, in order to adjust analyses accordingly. For a total of 26 demographic and clinical variables, marginal effects were quantified on microbiome community-level diversity. Faecal and salivary microbiome richness (as a proxy for alpha diversity) was not univariately associated with any tested variable, nor to PDAC status, when accounting for the most common PDAC risk factors and applying a false discovery rate (FDR) threshold of 0.05.

Microbiome community composition, in contrast, varied with age at diagnosis (PERMANOVA on between-sample Bray-Curtis dissimilarities, R2=0.01, Benjamini-Hochberg-corrected p=0.03), diabetes (R2=0.01, p=0.04), and jaundice status (R2=0.02, p=0.009) in faeces, and with aspirin/paracetamol usage (R2=0.02, p=0.04) in saliva, albeit at very low effect sizes. Even though cases and controls were matched for age and sex, these factors were included as strata for subsequent analyses. Under such adjustment, subject disease status was mildly but statistically significantly associated with community composition in faeces (R2 = 0.02, p= 0.001), but not in saliva (R2=0.01, p=0.5). Indeed, the faecal microbiome composition of PDAC patients differed from that of both control (R2=0.02, p≤0.0001) and CP subjects (R2=0.02, p=0.003), albeit likewise at very small effect sizes.

### Example 2. High-accuracy metagenomic classifiers capture specific faecal microbiome signatures in patients with PDAC.

The inventors have identified a model of 10 species (*Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii, and Bacteroides finegoldii)* that discriminates PDAC cases and controls with high accuracy in the study population (Area Under the Receiver Operator Curve, AUROC=0.81).

The most prominent positive marker species in the model were *Methanobrevibacter smithii, Alloscardovia omnicolens, Veillonella atypica* and *Bacteroides finegoldii.* None of the 25 demographic and epidemiological variables describing the study population were selected as predictive features by the model, and the microbiome signature was more informative than any other feature. Further, none of these variables were individually associated with the microbial species represented in the model, ruling them out as potential confounders. This indicates that the classifier captured a diagnostic gut microbiome signature of PDAC that is likely independent of other disease risk factors and potential confounders.

An analogous model built to discern CP patients from controls had no predictive power (AUROC = 0.5) consistent with the observation that these groups were compositionally largely indistinguishable. However, a faecal model to distinguish PDAC patients from CP cases performed better with an AUC of 0.75, but model robustness was limited by the low sample size in the CP group. It was further explored predictive associations at the higher resolution of functional microbiome profiles. Models based on the abundances of KEGG modules achieved an accuracy of up to AUROC=0.74, but feature selection was likewise not robust across validation folds, as a consequence of fitting a high number of variables (modules) against a limited set of samples.

When at least one of the following species: *Faecalibacillus intestinalis, Streptococcus oralis, Streptococcus gordonii*/*cristatus, Romboutsia timonensis, Ruminococcus callidus, Atopobium parvulum, Fusobacterium periodonticum, Fusobacterium hwasookii*/*nucleatum, Phascolarctobacterium faecium, Dialister pneumosintes, Prevotella sp. CAG:279* [meta-mOTU-v2.5 12279] and *Duodenibacillus massiliensis* were added to the model its accuracy was improved. When all these 12 species were added to the model, the discrimination of PDAC cases and controls showed higher accuracy in the study population (Area Under the Receiver Operator Curve, AUROC=0.85 (Fig.2).

### Combination of fecal microbiome data with CA19-9 results increase sensitivity.

Blood serum levels of the antigen CA19-9 are routinely used to monitor PDAC progress, but have also been suggested as a potential marker for PDAC early diagnosis, albeit with moderate reported sensitivity (0.80, 95%CI 0.72-0.86) and specificity (0.75, 95%CI 0.68-0.80). CA19-9 serum levels were available for a subset of 77 individuals (33/44 CTRs & 44/57 PDAC cases) in our Spanish population. Given that CA19-9 is directly secreted by tumors, it was hypothesised that the readouts provided by CA19-9 serum levels and by the microbiome classifiers were complementary, and that their combination could improve the accuracy of PDAC prediction. Indeed, accounting for CA19-9 increased the accuracy of our model from AUROC=0.81 to 0.91, driven mostly by an increase in sensitivity. Also, when the 22 species model was amended with CA19-9 information, the increase in accuracy went from AUC= 0.85 to 0.93 (Fig. 2). There was no significant bias towards higher CA19-9 levels in later disease stages. The Spanish study population included 25 PDAC patients in early disease stages (T1, T2) and 32 subjects in later stages (T3, T4). Disease stage did not affect the performance of either microbiome-based model; in particular, recall was not biased towards later stages.

### Example 3. Performance of metagenome-based classifiers generalizes to independent validation cohorts.

To assert that the observed microbiome signatures generalize beyond the focal Spanish study population, the models were next challenged in two validation scenarios. First, prediction accuracy was tested in an independent study population of 44 PDAC patients and 32 matched controls, recruited from two hospitals in Erlangen and Frankfurt a.M., Germany, with the samples being processed identically to the Spanish population. On this German validation population, our model validated with comparable or indeed superior accuracies to the training population, both with and without complementation by CA19-9 levels, and with similar trends across disease stages.

Next, to confirm that the metagenomic classifiers captured PDAC-specific signatures, rather than unspecific, more general disease-associated variation, they were further validated against independent, external metagenomic datasets on various health conditions. In total, 5,776 publicly available gut metagenomes from 25 studies across 18 countries were classified, including subjects suffering from chronic pancreatitis (CP, this study), type 1 (T1D) or type 2 diabetes (T2D), colorectal cancer (CRC), breast cancer (BRCA), liver diseases (LD), non-alcoholic fatty liver disease (NAFLD), including Crohn's disease (CD) and ulcerative colitis (UC), as well as healthy controls.

When tuned to 90% specificity (allowing for 10% false positive predictions) in the focal Spanish study population, our model showed a recall of 60% of PDAC cases in the Spanish population and 21% in the German validation population (with 0% FPR), and up to 83% when complemented with information on CA19-9 levels (available for 8/32CTRs & 43/44 PDAC cases in German cohort). Model disease specificity, however, was limited, with predictions of PDAC state for 6% of control subjects on average across all external datasets. Most of these false positive calls were observed in one Chinese population of patients suffering from liver cirrhosis which shares some physiological characteristics with impaired pancreas function. The effect was likely attributable in part to technical and demographic effects between studies. Indeed, it was noted that subjects in this Chinese study population was significantly younger than our populations (50±11y for Qin_2014; 70±12y for our Spanish population). This age effect was systematic: across all validation sets, PDAC prediction scores were associated with subject age (ANOVA p=0.007; ρSpearman = 0.16), as well as with subject sex (p<10-6;) and sequencing depth (p=0.0008; ρSpearman = 0.1).

### Example 4. Kit for the detection/quantification of microbiome species in faecal samples

A quantitative PCR (qPCR) based non-invasive, rapid and low-cost Microbial Abundance-based Stool Test (MAST) is designed to target PDAC-related changes based on species *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii, and Bacteroides finegoldii* in faecal samples, optionally also based on at least one of the additional species *Faecalibacillus intestinalis, Streptococcus oralis, Streptococcus gordonii*/c*ristatus, Romboutsia timonensis, Ruminococcus callidus, Atopobium parvulum, Fusobacterium periodonticum, Fusobacterium hwasookii*/*nucleatum, Phascolarctobacterium faecium, Dialister pneumosintes, Prevotella sp. CAG:279 [meta-mOTU-v2.5 12279]* and *Duodenibacillus massiliensis.* Specific primers for said species are designed and optionally also specific probes for each pair of specific primers are designed for the qPCR. Also, the kit comprises means for quantifying antigen CA19-9 in serum samples.

We use the kit directly on patient fecal samples. Rapid extraction of DNA from feces is followed by parallel targeted qPCR quantification of the species described above, based on sequence probes designed and vetted for sensitivity and specificity. We have approximated total DNA per sample by using qPCR quantification of 16S rRNA gene copies, as a baseline for species abundances. A universal primer set for the 16S rRNA gene which is suitable for qPCR method is used to normalize cycle threshold (Ct) values. A SPUD assay is implemented as an inhibitor indicator. We use a plate calibrator to detect pipetting errors caused by manual handling or the liquid handler process. To assess the discriminative power of each species, we first calculate delta Ct (dCt) value based on the difference between a given species' Ct value and 16S rRNA gene. Since MAST and CA19-9 tests capture different types of signatures that complement each other as shown in Fig. 2 and Fig 3, the combination increases overall specificity of PDAC detection.

### Materials and Methods

### Sample processing.

Faecal and salivary samples were thawed on ice, aliquoted and genomic DNA was extracted using the Qiagen Allprep PowerFecal DNA/RNA kit as per the manufacturer's instructions (Qiagen, Hilden, Germany). All samples were randomly assigned to extraction batches. To account for potential bacterial contamination of extraction, PCR and sequencing kits, we included negative controls (extraction blanks) with each tissue DNA extraction batch.

### Metagenome data processing.

Metagenomic data was processed using established workflows in NGLess v0.7.1. Raw reads were quality trimmed (≥45bp at Phred score ≥25) and filtered against the human genome (version hg19, mapping at ≥90% identity across ≥45bp). The resulting filtered reads were mapped (≥97% identity across ≥45bp) against the representative genomes of 5,306 species-level genome clusters obtained from the proGenomes database v2.

Taxonomic profiles were obtained using the mOTU profiler v2.5 (75) 233 and filtered to retain only species observed at a relative abundance ≥10^-5 in ≥2% of samples. Gene functional profiles were obtained from GMGCv1 mappings (http://gmge.embl.de/), by summarising read counts from eggNOG v4.5 (76) annotations to orthologous groups and KEGG modules. Features with a relative abundance of ≥10^-5 in ≥15% of samples were retained for further analyses.

### Multivariable statistical modeling and model evaluation.

In order to train multivariable statistical models for the prediction of pancreatic cancer, we first removed taxa with low overall abundance and prevalence (abundance cutoff: 0.001). Then, features were normalized by log10-transformation (to avoid infinite values from the logarithm, a pseudo-count of 1e-05 was added to all values) followed by standardization as centered log-ratio (log.clr). Data were randomly split into test and training sets in a 10 times repeated 10 fold cross validation. For each test fold, the remaining folds were used as training data to train an L1-regularized (LASSO) logistic regression model using the implementation within the LiblineaR R package v2.10. The trained model was then used to predict the left-out test set and finally, all predictions were used to calculate the Area Under the Receiver-Operating-Characteristics curve (AUROC).

In a second approach, features were filtered within the cross-validation (that is, for each training set) by first calculating the single-feature AUROC and then removing features with an AUROC <0.5, thereby selecting features enriched in PDAC.

In order to combine the predictions from the microbiome-based machine learning models with the CA19-9 marker, the coded CA19-9 marker (1 for positive, 0 for negative or NA) was added to the mean predictions from the repeated cross-validation runs, resulting in an OR combination. Alternatively, the AND combination was calculated by multiplying the predictions with the CA19-9 marker. ROC curves and AUROC values were calculated for both combinations using the pROC R package v1.15.

The trained ES metagenomic classifiers for PDAC were then applied to DE dataset after applying a data normalization routine which selects the same set of features and uses the same normalization parameters (for example the mean of a feature for standardization by using the frozen normalization functionality in SIAMCAT) as in the normalization procedure from the ES pancreatic cancer dataset. For this analysis, the cutoff for the predictions was set to a false positive rate of 10% among controls in the initial ES PDAC study population.

## Claims

1. A method for diagnosing pancreatic cancer in a subject comprising:
a. determining the abundance of at least the following Microbiome species: *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii, and Bacteroides finegoldii* in a faecal sample from said subject, and:
b. identifying the subject as:
i. having pancreatic cancer when the profile of abundance for the analysed species corresponds to a profile of abundance of the analysed species of a pancreatic cancer reference group, or
ii. not having pancreatic cancer when the profile of abundance for the analysed species corresponds to a profile of abundance of the analysed species of a reference group not having pancreatic cancer.

2. The method according to claim 1, further comprising analysing the abundance of at least one of the following species: *Faecalibacillus intestinalis, Streptococcus oralis, Streptococcus gordonii*/*cristatus, Romboutsia timonensis, Ruminococcus callidus, Atopobium parvulum, Fusobacterium periodonticum, Fusobacterium hwasookii*/*nucleatum, Phascolarctobacterium faecium, Dialister pneumosintes, Prevotella sp. CAG:279 [meta-mOTU-v2.5 12279]* and *Duodenibacillus massiliensis* in said faecal sample.

3. The method of according to any one of claims 1 or 2, consisting essentially in:
a. analysing the abundance of at least the following Microbiome species: *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii and Bacteroides finegoldii,* in said faecal sample and:
b. identifying the subject as:
i. having pancreatic cancer when the profile of abundance for the analysed species corresponds to a profile of abundance of the analysed species of a pancreatic cancer reference group, or
ii. not having pancreatic cancer when the profile of abundance for the analysed species corresponds to a profile of abundance of the analysed species of a reference group not having pancreatic cancer.

4. The method of according to any one of claims 1 to 3, further comprising analysing the levels of carbohydrate antigen (CA) 19-9 in a serum sample from said subject.

5. The method of according to any one of claims 1 to 4, wherein the subject is human.

6. The method of according to any one of claims 1 to 5, wherein pancreatic cancer is in an early stage or a late stage of said pancreatic cancer.

7. The method of according to any one of claims 1 to 6, wherein step (a) is performed by whole-genome sequencing, qPCR, ddPCR, 16S rRNA amplicon sequencing, or a combination thereof.

8. The method according to claim 7, wherein whole genome sequencing is performed using shotgun metagenomic sequencing.

9. The method of according to any one of claims 1 to 8, wherein the pancreatic cancer is pancreatic ductal adenocarcinoma (PDAC).

10. A kit of parts comprising specific primers and optionally also specific probes for the detection/quantification of the microbial presence/abundance of the species *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii, and Bacteroides finegoldii.*

11. The kit according to claim 10, further comprising specific primers and optionally also specific probes for the detection/quantification of the microbial presence/abundance of at least one of species *Faecalibacillus intestinalis, Streptococcus oralis, Streptococcus gordonii*/*cristatus, Romboutsia timonensis, Ruminococcus callidus, Atopobium parvulum, Fusobacterium periodonticum,*
*Fusobacterium hwasookii*/*nucleatum, Phascolarctobacterium faecium, Dialister pneumosintes, Prevotella sp. CAG:279 [meta-mOTU-v2.5 12279]* and *Duodenibacillus massiliensis.*

12. The kit according to any one of claims 10 or 11, further comprising specific primers and optionally also specific probes for the detection/quantification of at least one control species.

13. The kit of parts according to any one of claims 10 to 12, further comprising means for quantifying antigen CA19-9 in a serum sample.

14. Use of the kit of any one of claims 10 to 13 for the diagnosis of pancreatic cancer, preferably of PDAC.

15. Use of the combination of species *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii, and Bacteroides finegoldii,* and optionally at least one of species *Faecalibacillus intestinalis, Streptococcus oralis, Streptococcus gordonii*/*cristatus, Romboutsia timonensis, Ruminococcus callidus, Atopobium parvulum, Fusobacterium periodonticum, Fusobacterium hwasookii*/*nucleatum, Phascolarctobacterium faecium, Dialister pneumosintes, Prevotella sp. CAG:279 [meta-mOTU-v2.5 12279]* and *Duodenibacillus massiliensis,* as biomarkers for the diagnosis and/or monitoring of pancreatic cancer, preferably PDAC.

## Patentansprüche

1. Verfahren zur Diagnose von Bauchspeicheldrüsenkrebs bei einem Subjekt, umfassend:
a. Bestimmung der Abundanz von mindestens den folgenden Mikrobiom-Spezies in einer Stuhlprobe des genannten Subjekts: *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii* und *Bacteroides finegoldii,* und:
b. Identifizierung des Subjekts als:
i. an Bauchspeicheldrüsenkrebs erkrankt, wenn das Abundanzprofil der analysierten Spezies einem Abundanzprofil der analysierten Spezies einer Referenzgruppe mit Bauchspeicheldrüsenkrebs entspricht, oder
ii. nicht an Bauchspeicheldrüsenkrebs erkrankt, wenn das Abundanzprofil der analysierten Spezies einem Abundanzprofil der analysierten Spezies einer Referenzgruppe ohne Bauchspeicheldrüsenkrebs entspricht.

2. Verfahren nach Anspruch 1, ferner umfassend die Analyse der Abundanz von mindestens einer der folgenden Spezies in der genannten Stuhlprobe: *Faecalibacillus intestinalis, Streptococcus oralis, Streptococcus gordonii*/*cristatus, Romboutsia timonensis, Ruminococcus callidus, Atopobium parvulum, Fusobacterium periodonticum, Fusobacterium hwasookii*/*nucleatum, Phascolarctobacterium faecium, Dialister pneumosintes, Prevotella sp. CAG:279 [meta-mOTU-v2.5 12279]* und *Duodenibacillus massiliensis.*

3. Verfahren nach einem der Ansprüche 1 oder 2, im Wesentlichen bestehend aus:
a. Analyse der Abundanz von mindestens den folgenden Mikrobiom-Spezies in der genannten Stuhlprobe: *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii* und *Bacteroides finegoldii,*und:
b. Identifizierung des Subjekts als:
i. an Bauchspeicheldrüsenkrebs erkrankt, wenn das Abundanzprofil der analysierten Spezies einem Abundanzprofil der analysierten Spezies einer Referenzgruppe mit Bauchspeicheldrüsenkrebs entspricht, oder
ii. nicht an Bauchspeicheldrüsenkrebs erkrankt, wenn das Abundanzprofil der analysierten Spezies einem Abundanzprofil der analysierten Spezies einer Referenzgruppe ohne Bauchspeicheldrüsenkrebs entspricht.

4. Verfahren nach einem der Ansprüche 1 bis 3, ferner umfassend die Analyse der Konzentration an Kohlenhydrat-Antigen (CA) 19-9 in einer Serumprobe des genannten Subjekts.

5. Verfahren nach einem der Ansprüche 1 bis 4, wobei das Subjekt ein Mensch ist.

6. Verfahren nach einem der Ansprüche 1 bis 5, wobei der Bauchspeicheldrüsenkrebs in einem Früh- oder Spätstadium des genannten Bauchspeicheldrüsenkrebses vorliegt.

7. Verfahren nach einem der Ansprüche 1 bis 6, wobei Schritt (a) durch Ganzgenomsequenzierung, qPCR, ddPCR, 16S rRNA-Amplikonsequenzierung oder eine Kombination derselben durchgeführt wird.

8. Verfahren nach Anspruch 7, wobei die Ganzgenomsequenzierung mittels Schrotschuss-Metagenomsequenzierung durchgeführt wird.

9. Verfahren nach einem der Ansprüche 1 bis 8, wobei der Bauchspeicheldrüsenkrebs ein duktales Adenokarzinom des Pankreas (PDAC) ist.

10. Kit aus Einzelkomponenten, bestehend aus spezifischen Primern und wahlweise spezifischen Sonden zur Detektion/Quantifizierung des Vorhandenseins/der Abundanz von Mikroorganismen der Spezies *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii* und *Bacteroides finegoldii.*

11. Kit nach Anspruch 10, ferner umfassend spezifische Primer und wahlweise spezifische Sonden zur Detektion/Quantifizierung des Vorhandenseins/der Abundanz von Mikroorganismen mindestens einer der Spezies *Faecalibacillus intestinalis, Streptococcus oralis, Streptococcus gordonii*/*cristatus, Romboutsia timonensis, Ruminococcus callidus, Atopobium parvulum, Fusobacterium periodonticum, Fusobacterium hwasookii*/*nucleatum, Phascolarctobacterium faecium, Dialister pneumosintes, Prevotella sp. CAG:279 [meta-mOTU-v2.5 12279]*und *Duodenibacillus massiliensis.*

12. Kit nach Anspruch 10 oder 11, ferner umfassend spezifische Primer und wahlweise spezifische Sonden zur Detektion/Quantifizierung von mindestens einer Kontrollspezies.

13. Kit aus Einzelkomponenten nach einem der Ansprüche 10 bis 12, ferner umfassend Mittel zur Quantifizierung des Antigens CA19-9 in einer Serumprobe.

14. Verwendung des Kits nach einem der Ansprüche 10 bis 13 zur Diagnose von Bauchspeicheldrüsenkrebs, insbesondere von PDAC.

15. Verwendung einer Kombination der Spezies *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/ *intermedius, Methanobrevibacter smithii* und *Bacteroides finegoldii* sowie wahlweise mindestens einer der Spezies *Faecalibacillus intestinalis, Streptococcus oralis, Streptococcus gordonii*/*cristatus, Romboutsia timonensis, Ruminococcus callidus, Atopobium parvulum, Fusobacterium periodonticum, Fusobacterium hwasookii*/*nucleatum, Phascolarctobacterium faecium, Dialister pneumosintes, Prevotella sp. CAG:279 [meta-mOTU-v2.5 12279]* und *Duodenibacillus massiliensis* als Biomarker zur Diagnose und/oder Verlaufskontrolle von Bauchspeicheldrüsenkrebs, insbesondere PDAC.

## Revendications

1. Procédé pour le diagnostic du cancer du pancréas chez un sujet comprenant :
a. déterminer l'abondance au moins des espèces de microbiome suivantes : *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/*intermedius, Methanobrevibacter smithii,* et *Bacteroides finegoldii* dans un échantillon fécal dudit sujet, et ;
b. identifier le sujet comme :
i. ayant du cancer du pancréas lorsque le profil d'abondance pour les espèces analysées correspond à un profil d'abondance des espèces analysées d'un groupe de référence de cancer du pancréas, ou
ii. n'ayant pas de cancer du pancréas lorsque le profil d'abondance pour les espèces analysées correspond à un profil d'abondance des espèces analysées d'un groupe de référence n'ayant pas de cancer du pancréas.

2. Procédé selon la revendication 1, comprenant en outre l'analyse de l'abondance d'au moins une des espèces suivantes : *Faecalibacillus intestinalis, Streptococcus oralis, Streptococcus gordonii*/*cristatus, Romboutsia timonensis, Ruminococcus callidus, Atopobium parvulum, Fusobacterium periodonticum, Fusobacterium hwasookiilnucleatum, Phascolarctobacterium faecium, Dialister pneumosintes, Prevotella sp.* CAG : 279 [meta-mOTU-v2.5 12279] et *Duodenibacillus massiliensis* dans ledit échantillon fécal.

3. Procédé selon l'une quelconque des revendications 1 ou 2, qui consiste essentiellement à :
a. analyser l'abondance au moins des espèces de microbiome suivantes : *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/*intermedius, Methanobrevibacter smithii* et *Bacteroides finnegoldii,* dans ledit échantillon fécal et :
b. identifier le sujet comme :
i. ayant du cancer du pancréas lorsque le profil d'abondance pour les espèces analysées correspond à un profil d'abondance des espèces analysées d'un groupe de référence de cancer du pancréas, ou
ii. n'ayant pas de cancer du pancréas lorsque le profil d'abondance pour les espèces analysées correspond à un profil d'abondance des espèces analysées d'un groupe de référence n'ayant pas de cancer du pancréas.

4. Procédé selon l'une quelconque des revendications 1 à 3, comprenant en outre l'analyse des niveaux d'antigène glucidique (CA) 19-9 dans un échantillon de sérum dudit sujet.

5. Procédé selon l'une quelconque des revendications 1 à 4, dans lequel le sujet est un être humain.

6. Procédé selon l'une quelconque des revendications 1 à 5, dans lequel le cancer du pancréas est à un stade précoce ou un stade tardif dudit cancer du pancréas.

7. Procédé selon l'une quelconque des revendications 1 à 6, dans lequel l'étape (a) est réalisée par séquençage du génome entier, qPCR, ddPCR, séquençage d'ARNr 16S, ou une combinaison de ceux-ci.

8. Procédé selon la revendication 7, dans lequel le séquençage du génome entier est réalisé en utilisant un séquençage aléatoire métagénomique.

9. Procédé selon l'une quelconque des revendications 1 à 8, dans lequel le cancer du pancréas est un adénocarcinome de conduits pancréatiques (PDAC).

10. Kit de composants comprenant des amorces spécifiques et optionnellement en outre des sondes spécifiques pour la détection/quantification de la présence/abondance microbienne des espèces *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/*intermedius, Methanobrevibacter smithii* et *Bacteroides finegoldii.*

11. Kit selon la revendication 10, comprenant en outre des amorces spécifiques et optionnellement en outre des sondes spécifiques pour la détection/quantification de la présence/abondance microbienne d'au moins une des espèces *Faecalibacillus intestinalis, Streptococcus oralis, Streptococcus gordonii*/*cristatus, Romboutsia timonensis, Ruminococcus callidus, Atopobium parvulum, Fusobacterium periodonticum, Fusobacterium hwasookiilnucleatum, Phascolarctobacterium faecium, Dialister pneumosintes, Prevotella sp.* CAG : 279 [meta-mOTU-v2.5 12279] et *Duodenibacillus massiliensis.*

12. Kit selon l'une quelconque des revendications 10 ou 11, comprenant en outre des amorces spécifiques et optionnellement en outre des sondes spécifiques pour la détection/quantification d'au moins une espèce de contrôle.

13. Kit de composants selon l'une quelconque des revendications 10 à 12, comprenant en outre des moyens pour la quantification de l'antigène CA19-9 dans un échantillon de sérum.

14. Utilisation du kit selon l'une quelconque des revendications 10 à 13 pour le diagnostic de cancer du pancréas, de préférence de PDAC.

15. Utilisation de la combinaison des espèces *Alloscardovia omnicolens, Veillonella atypica, Veillonella dispar, Veillonella parvula, Veillonella sp.* [meta-mOTU-v2.5 13135], *Butyrivibrio crossotus, Faecalibacillus faecis, Streptococcus anginosus*/*intermedius, Methanobrevibacter smithii,* et *Bacteroides finegoldii,* et optionnellement d'au moins une des espèces *Faecalibacillus intestinalis, Streptococcus oralis, Streptococcus gordonii*/*cristatus, Romboutsia timonensis, Ruminococcus callidus, Atopobium parvulum, Fusobacterium periodonticum, Fusobacterium hwasookiilnucleatum, Phascolarctobacterium faecium, Dialister pneumosintes, Prevotella sp.* CAG : 279 [meta-mOTU-v2.5 12279] et *Duodenibacillus massiliensis,* comme biomarqueurs pour le diagnostic et/ou la surveillance de cancer du pancréas, de préférence de PDAC.
